# EUROPEAN PATENT APPLICATION

(11) **EP 0 552 368 A1**
(43) Date of publication of application: **28.07.1993**
(21) Application number: 91919256.7
(22) Date of filing: 09.10.1991
(51) Int. Cl.: C07K 3/12, C07K 1/14, C07K 17/00, C07C 271/14, C07C 317/28, C07C 323/65, C07D 207/46, C07K 3/10, C07K 3/08, C07K 1/04

(54) **PROCESS FOR PURIFYING SYNTHETIC PEPTIDE, AND LINKER AND LINKER-COMBINING SOLID-PHASE CARRIER USED IN SAID PROCESS**

(30) Priority: 09.10.1990 JP 269640/90
(71) Applicant: NIHON MILLIPORE KABUSHIKI KAISHA, Shinagawa-ku, Tokyo (JP); FUNAKOSHI, Susumu, Kyoto-shi, Kyoto 606 (JP)
(72) Inventor: FUNAKOSHI, Susumu, Kyoto-shi, Kyoto 606 (JP); FUKUDA, Hiroyuki, Toyonaka-shi, Osaka 561 (JP)
(74) Representative: Henkel, Feiler, Hänzel & Partner
(86) International application number: JP9101379
(87) International publication number: WO9206107

(57) **Abstract**

A process for purifying a peptide synthesized by the solid phase method with high accuracy and high yield in one step, which comprises separating a target amino-terminated maturation peptide from the reaction system containing both the maturation peptide and other end-capped nonmaturation peptides formed in the peptide synthesis according to the solid phase method by immobilizing the maturation peptide chemico-selectively on the carrier by means of a function-terminated linker.

## Description

### Background of the Invention

### (Industrial Utility)

The present invention relates to a method for the purification of synthesized peptides, and relates to a linker and solid-phase ligand for bonding the linker for this purification method. More particularly the invention relates to a method for performing a one-step purification of peptides, being synthesized by a solid-phase method, with high accuracy and high yield, and relates to the linker and the solid-phase ligand for bonding the linker used for this purification.

### (Prior Art)

Peptides or proteins are biological molecules existing normally in organis. The elucidation of physiological activities and of mechanisms of the biological molecules are interested much in the fields of biochemistry, physiology and medicine. The synthesis of peptides or proteins having specific amino acid sequences has recently been made actively with use of automated peptide synthesizers. Studies in the above mentioned fields are expected to show sharp progress, if the synthesized peptides or proteins having specific amino acid sequences are provided with higher purities. However, present peptide synthesis methods produce relative large volume of impurities as well as a target compound. Therefore the most pending objective of the solid-phase peptide synthesis method is to recover the target peptide alone from impurities with high speed and high yield.

Gel filtration, high-performance liquid chromatography, or the combination thereof is used at present for the purification of peptides or proteins synthesized by a solid-phase method (R. B. Merrifield, J. Am. Chem. Soc., 85, 2149 (1963)). In respect with some kinds of special peptides, the affinity chromatography may be an effective purification method, but not perfect one. The reason is that some of various amino acid deleted peptides may have an affinity (even a low degree) for the supports used in affinity chromatography, the amino acid deleted peptides being synthesized as impurities during the solid-phase synthesis as part of the resultant peptide mixture.

The solid-phase synthesis of peptides is made by a step-wise elongation. For example, in case of condensation of a 50 residue peptide with the condensation reaction yield of 99%, the theoretical synthetic yield reaches to 60%. Condensation reaction yield over 99% can not be always obtained since the condensation reaction depends on the sequence of peptides. As a result, amino acid deleted peptides pile up as impurities by incomplete condensation reactions.

A capping by acetic anhydride is performed after every condensation reaction to terminate further elongation of peptide chains of a non-target sequence and to avoid further production of amino acid deleted peptides. The procedure results in that only the peptide having a target amino acid sequence has an amino group at its N-terminus after the coupling of the final amino acid.

Several reports on the purification methods with use of the N-terminus amino group have been published. (See, for example, R. Gamble, R. Garner, and G. T. Young, Nature (London), 217, 241 (1968); K. Suzuki Y. Sasaki, and N. Endo, Chem. Pharm. Bull., 24, 1 (1976); D. S. Kemp and D. G. Roberts, Tetrahedoron Lett., 4269 (1975); T. Weiland, C. Birr, and H. Wissenbach, Angew. Chem., Int. Ed., Engl., 8, 764 (1969), H. Wissman and R. Geiger, Angew. Chem., Int. Ed., Engl., 9, 908 (1970); R. B. Merrifield and A. E. Bach, J. Org. Chem. 43, 4808 (1975); T. J. Lobl, R. M. Deibel, and A. W. Yen, Anal. Biochem., 170, 502 (1988); H. Ball, C. Grecian, S. B. H. Kent, and P. Mascagni, in "Peptides", J. E. Rivier and G. R. Marshall, Eds., ESCOM, Leiden 1990 pp435). However, none of these methods has been able to achieve effective one-step separation, instead they have required complicated processes.

Another method has been developed in which the target peptide alone is absorbed to a phenyl-mercury column by attaching cysteine-methionine to the N-terminus of the synthesized peptide, and using the SH group of the cysteine. Subsequent to the separation, the methioin-peptide bond is cleaved by BrCN to yeild the target peptide. (D. E. Krieger, B. W. Erikson, and R. B. Merrifield, in Proc. Nat. Acad. Sci. U. S. A., 73, 3160 (1976)) However, The method has a limitation of being not applicable to peptides containing methionine.

### Summary of the Invention

An objective of this invention is to offer a method for purifying peptides made by solid-phase synthesis, and a linker and a solid-phase ligand for bonding the linker used therein so as to enable, performing one-step purification with high-accuracy and high-yield.

The above objective is achieved by a purification method of synthesized peptide in which a target mature peptide having an amino group at the terminus is chemically and selectively isolated from mixture of the mature target peptide and the end-capped immature amino acid deleted peptides by immobilization to the solid-phase ligand via the linker having a functional group at the terminus.

The above objective is also achieved by a purification method of synthesized peptide comprising:
a) after adding the final amino acid for a solid-phase peptide synthesis, adding a linker to the solid-phase to which both a mature target peptide with an amino group at a terminus and immature peptides end-capped by an acylating agent such as anhydrous acetic acid or anhydrous propionic acid have been bound, thereby selectively modifying the mature peptide with the linker, wherein the linker has at one terminus a functional group which is able to form a bond with the N-terminus of the mature peptide, the bond being stable to the final deprotection reaction under acidic condition and being specifically cleaved under another condition, and wherein the opposite terminus site of the linker is able to be converted into another active functional group by the deprotection reaction;
b) dissociating the mature peptide and the immature peptides from the solid-phase support by the deprotection reaction performed as the final process of the peptide synthesis;
c) contacting the resultant freed peptide mixture of above step b) with a solid-phase ligand, and thereby selectively immobilizing the mature peptide to the solid-phase ligand via the linker, wherein the solid-phase ligand has a compound immobilized thereon and which having a functional group capable of forming a stable bond with the functional group of the opposite terminus of the linker produced by the deprotection reaction; and
d) exposing the mature peptide immobilized ligand of above step c) to the other condition under which the bond between the mature peptide and the linker is selectively cleaved, thereby separating the mature peptide from the linker bound ligand.
   A preferred embodiment of the method according to the present invention is to isolate the target mature peptide by the following steps:
e) after introducing the final amino acid for a solid-phase peptide synthesis, adding a linker to the solid-phase to which both a mature target peptide with an amino group at a terminus and immarture end-capped peptides have been bound, thereby selectively modifying the mature peptide with the linker, wherein the linker has at one terminus a functional group which is able to form a bond with the N-terminus of the mature peptide, the bond being stable to the final deprotection reaction under an acidic condition and also being specifically cleaved under an alkaline or basic condition, and wherein the opposite terminus site of the linker is able to be converted into an SH group by the deprotection reaction;
f) dissociating the mature peptide and the immature peptides from the solid-phase support by the deprotection reaction performed as the final process of the peptide synthesis;
g) contacting the resultant freed peptides mixture of above f) step with a solid-phase ligand, and thereby selectively immobilizing the mature peptide to the solid-phase ligand via the linker, wherein the solid-phase ligand has a compound of immobilized thereon and which having a functional group capable of forming a stable bond with the SH-group of the opposite terminus of the linker produced by the deprotection reaction; and
h) exposing the mature peptide immobilized ligand of above g) step to the alkaline condition under which the bond between the mature peptide and the linker is cleaved selectively, thereby separating the mature peptide from the linker-bound ligand.
   Another embodiment of the method according to the present invention is also to isolate the mature target peptide by the following steps:
i) after introducing the final amino acid for a solid-phase peptide synthesis, adding a linker to the solid-phase to which both a mature target peptide with an amino group at a terminus and immature end-capped peptides have been bound, thereby selectively modifying the mature pepetide, wherein the linker has at one terminus an alkoxycarbonate group which can form a urethane bond with the N-terminus of the target peptide, the urethane bond bieng stable to the final deprotection reaction under an acidic conditions and also being specifically cleaved under an basic condition, and wehrein the opposite terminus site of linker is able to be converted into a thiol group by the deprotection reaction;
j) dissociating the mature peptide and the immature peptides from the solid-phase support by the deprotection reaction performed as the final process of the peptide synthesis, while cleaving a thiol protecting group existing at the opposite terminus site of the linker in order to produce the thiol group;
k) contacting the resultant freed peptide mixture of above i) step with solid-phase ligand under a neutral condition, wherein the solid-phase ligand has a compound selected from the group consisting of iodo- and bromo-substituted alipathic carboxylic acids derivatives and immobilized thereon, thereby selectively immobilizing the mature peptide to the solid-phase ligand via the linker with a stable covalent bond, the covalent bond resulting from the deiodo- or debromo- reaction; and
l) exposing the mature peptide immobilized ligand of above k) step to the alkaline condition in order to effect the beta-elimination reaction on the bond between the mature target peptide and the linker, thereby cleaving the mature peptide from the linker-bound ligand.

A linker having the following structural formula (III) is preferably used for the method of this invention: 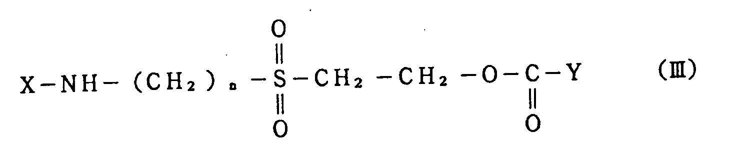
wherein n is an integral of one to four,
X is one of 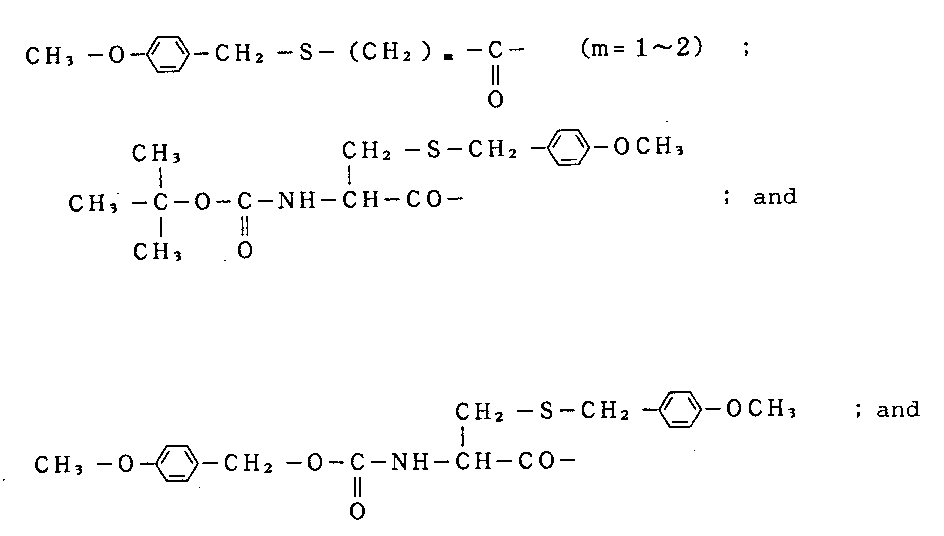
Y is one of

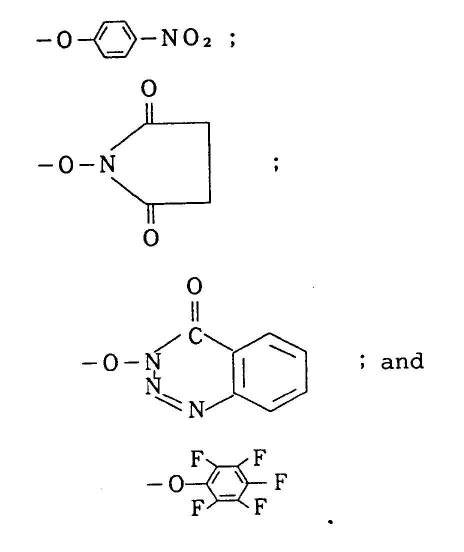

The above linkers can be easily synthesized from intermediates which are represented by the following structural formula (I) or intermediates by the following structural formula (II): 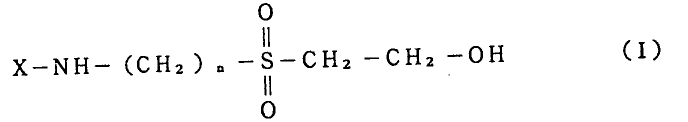
wherein n is an integral of one to four, X is one of H, hydrochloride thereof (H· HCl), 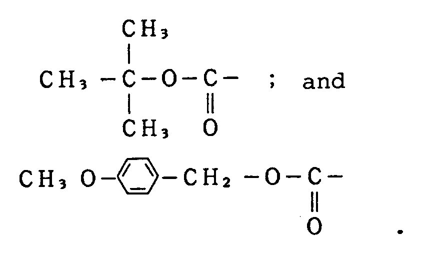 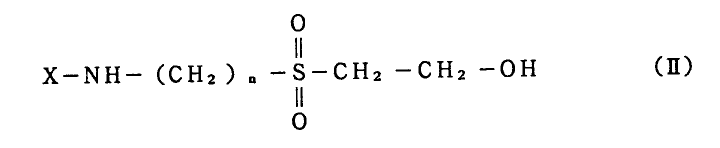
wherein n is an integral of one to four,
X is one of

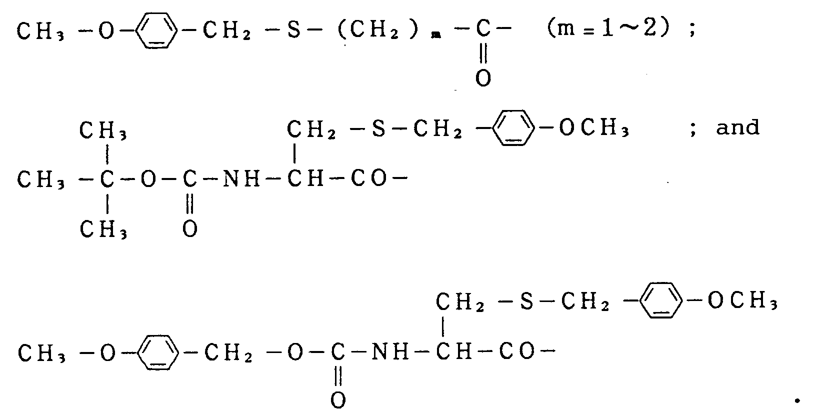

A solid-phase ligand for binding of linker, which is characterized by the property that molecular chains having one of the following structural formulae are bound to the surface thereof, is preferably used for the purification of this invention. 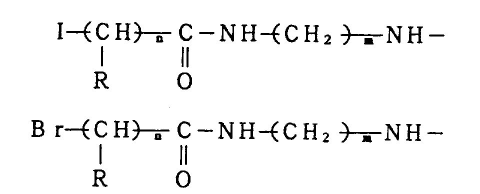
The inventors have studied diligently a method to separate rapidly and effectively the mature target peptide alone from the mixture of mature peptide and many immature peptides in the solid-phase peptide synthesis. Finally, we come to a conclusion that the method includes a selective modification of the mature peptide by a linker which has at one terminus a functional group capable of bonding with the N-terminus of the mature peptide. This bond is stable to the deprotection reaction under acidic condition in the post-treatment of the peptide synthesis process and is able to be cleaved specifically under another condition, and an opposite site of the linker is able to be converted into another active functional group by the deprotection reaction. When bonding the linker to the N-terminus of the mature peptide after the final amino acid coupling in the peptide synthesis is performed, the mature peptide alone has at its terminus the active functional group in the mature and immature peptide mixture of post-final deprotection reaction, the active functional group being due to the bound linker. When the mixture is contacted with another solid-phase ligand where a compound having the functional group capable of forming the stable bond with the active functional group of the mature peptide is immobilized thereon, this solid-phase ligand can selectively immobilize the mature peptide alone. This results in the separation of the target mature peptide from the mixture containing immature peptides. The target mature peptide may be cleaved from the linker-bound ligand, when the ligand immobilized the mature peptide is exposed to other conditions that specifically cleave the bond between the mature peptide and the linker.

The method of this invention can rapidly purify the target mature peptide prepared by the solid-phase synthesis with specific sequences, with high yield. Therefore, the method will make many contributions the elucidation of physiological activities and mechanism of peptides or proteins, and induce a sharp progress of the synthesis of peptides and proteins.

### Brief description of Drawings

Figure 1 is a flow chart showing peptide synthesis by F-moc solid-phase method and the following purification of the mature peptide;
Figure 2 is a flow chart showing process of peptide synthesis, the process of bonding of linker, and purification process with use of a linker-bound ligand;
Figure 3 is a flow chart showing reaction pathway of the synthesis of the linker;
Figure 4 is a flow chart showing another reaction pathway of the synthesis of the linker; and
Figure 5 is a flow chart showing other reaction pathway of the synthesis of the linker.

### Detailed Description of Invention

The present invention is hereby described in detail.

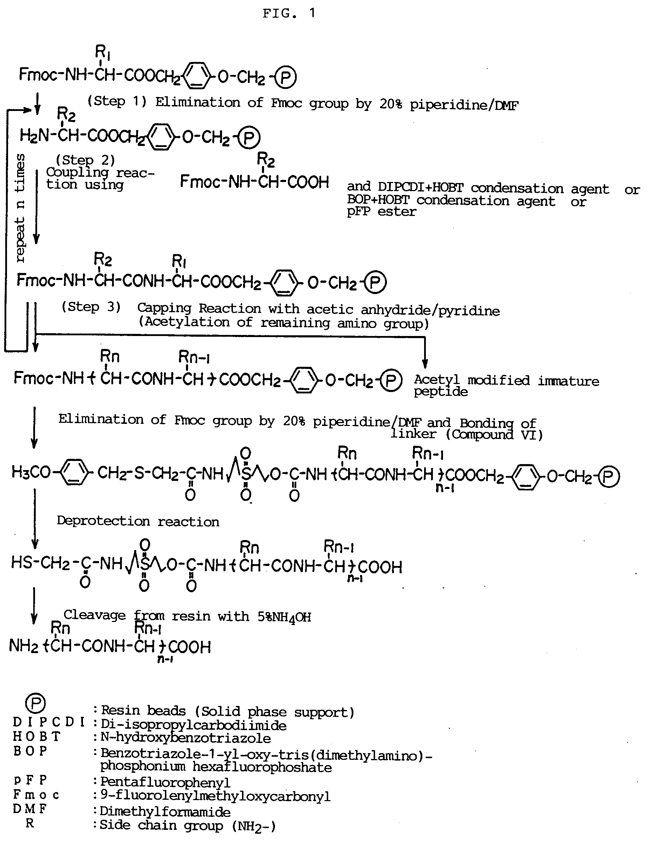

Figure 1 illustrates an embodiment of the method according to this invention for purifying peptides prepared by the F-moc (9-fluorenylmethyloxycarbonyl) method of solid-phase synthesis.

Peptide synthesis, as shown in Figure 1, is made by the repetition of step 1 to step 3 in the conventional way. Step 1 involves the following procedure : Many first amino residue 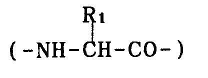
groups whose N-terminus is protected by F-moc group are bound to solid-phase support beads P; the support beads P are put into a reaction column of a peptide-synthesizer, where the mixed solution of 20% piperidine and dimethylformamide (DMF) is added in order to deproteoct N-terminal F-moc group.
Step 2 involves : N-terminal F-moc blocked second amino acid 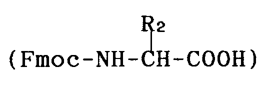
and a condensation agent is added to the column in order to bond the second amino residue group next to the first one bound to the support beads P. As a condensation agent, N,N'-di-isopropylcarbodiimide (DIPCD) + N-hydroxybenzotriazole (HOBT), Benzotriazole-1-yl-oxy-tris(dimethylamino)-phosphonium hexafluorophoshate (BOP) + HOBT, or Pentafluorophenylester of these amino acids + HOBT is used.
Step 3 involves : The chains of the first amino acid that are not bound to the second amino acid should not be elongated in further synthetic steps; the mixture of acetic anhydride/pyridine is added to the column to let the terminus of non-reacted chains end-cap by acetyl-bonding. On the support beads there are two types of peptide chains, i.e., one group is that of the first and the second amino acid residues bound together whose N-terminus is protected by an F-moc group, and the other is that the first amino residue whose N-terminus is bound to a acetyl group.

The process continues back to step 1. The N-terminal F-moc group of the chains where the first and the second amino residues are bound together is deprotected. As in step 2, the third amino acid and the condensation agent are added to let the third one bond to the second. The end-capping, as in step 3, is made to the chains where the third one is not bound. The stepping through from step 1 to step 3 repeats till targeted n groups of amino acids are bound.

After the coupling of the final amino acid, two types of peptides are bound to the solid support (solid-phase support beads P), namely, the mature target peptide having an amino group at the N-terminus, and the immature peptides having termini modified by an acetyl-group from the capping with acetic anhydride. The target peptide is mixed with many kinds of impurities.

Figure 1 describes the method to end-cap the terminus of immature peptides with an acetyl group. However, any kind of capping reagent can be used for the capping of the termini of immature peptides, if the capping is effective. As examples, the capping with propionyl group, 4-nitrophenyl group, 2,4-dinitrophenyl group, or 2,6-dinitrophenyl group is usable.

In the present invention for the purification of synthesized peptides, a qualified linker is added to the solid-phase support after the coupling of the last amino acid. The linker should have a functional group at one terminus, which forms with the N-terminus of the mature peptide a bond, such as urethane bond, which is stable under the acidic condition of the final deprotection reaction and cleavable specifically under other conditions such as basic conditions. The linker should also form an active functional group at the other terminus, when the deprotection reaction is performed as the final synthetic process.

The F-moc group (9-fluorolenylmethyloxycarbonyl) and the Msc group (methylsulphonylethyloxycarbonyl) are suitable as the amino protection groups stable to the final deprotection reaction performed under acidic conditions and deleted under basic conditions. These were developed respectively by Carpino et al., (L. A. Carpino and G. Y. Han, J. Am. Chem. Soc., 92, 5478(1970) and J. Org. Chem., 37, 3404 (1972)) and by Tesser et al., (G. I. Tesser and I. C. Balvert-Geers, Int. J. Peptide Protein Res., 7, 295 (1975)).

### Fmoc group:

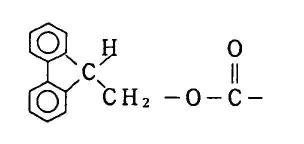

### Msc group :

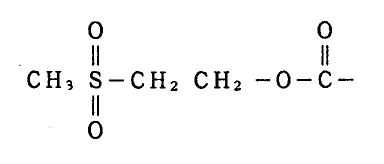

Both amino protection groups can be deleted in a short time, undergoing a β -elimination reaction by an alkaline treatment. Therefore a linker for the present invention can be preferably constructed based on these amino protection groups. The Msc group by Tesser et. al., is more available because of the ease of the synthesis and the length of its molecular chain. As the linker available for this invention, in a practical example, compounds represented by the structural formula (III) as shown below are preferable. 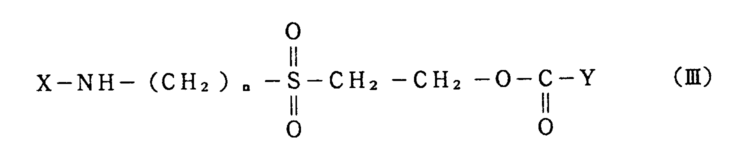
wherein n is an integral of one to four,
X is one of 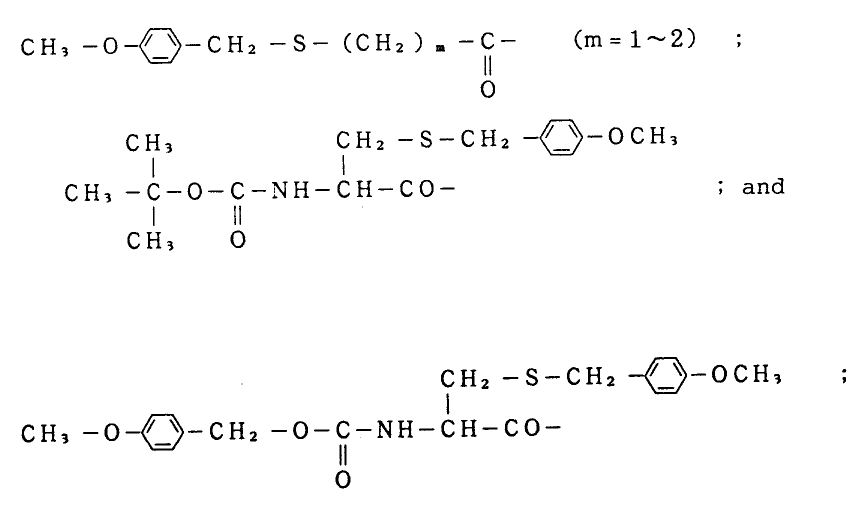
Y is one of

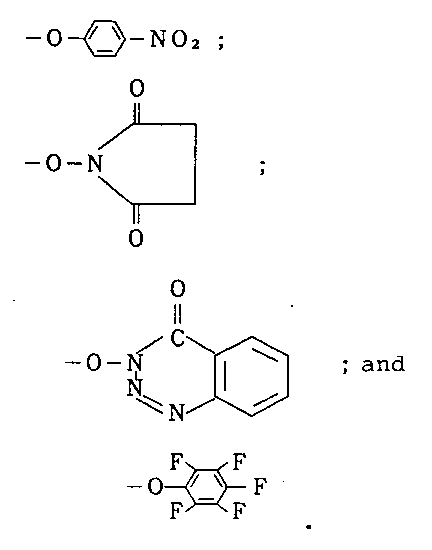

The linker comprises the Msc group as its basic structure, active alkoxycarbonate or allyloxycarbonate at one end which can be coupled to the N-terminal amino group of the peptide, and the thiol group (SH group) as an active functional group at the other end (SH group is in the state of being protected by a protection group). One end of the linker forms a bond with the N-terminus of the peptide. The bond is stable to any kind of acid used as a deprotection reagent in the deprotection process of solid-phase peptide synthesis, and is easily cleaved by an alkaline reagent causing a β -elimination reaction, such as 0.2N NaOH (50w/w% in methanol, 5 sec.) or 5w/w% NH₄OH (50w/w% in methanol, 5 min.). The SH group of the other end of the linker reacts with an iodo acetic acid derivative, permitting the formation of a stable covalent bond of thioester type under neutral or basic conditions.

The synthetic pathway to the linker represented by structural formula (III) is not subject to any limitation. As an example, the linker is preferably synthesized from the intermediate represented by the structural formula (I) involving the Msc group as a basic structure. 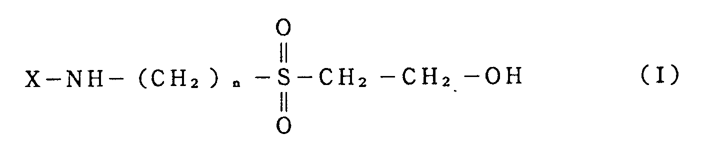
wherein n is an integral of one to four X is one of H, hydrochlolide thereof (H· HCl),

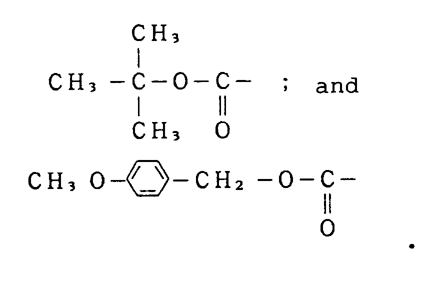

The hydroxyl group at one end of the intermediate represented by structural formula (I) can easily be converted to an active alkoxycarbonate which can couple with an amino group of a peptide or protein. On the other hand, an amino group or an amino group protected by a suitable protection group at the other terminus of the intermediate (I) can easily couple with the compound having another functional group, eg. compounds having a protected SH group.

An intermediate represented by structural formula (II) is a compound which may be obtained by introducing a thiol group protected with a protection group to the other terminal amino group of the intermediate represented by structural formula (I). 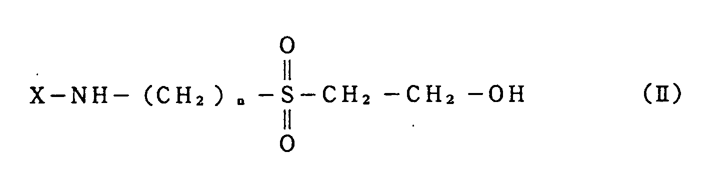
wherein n is an integral of one to four,
X is one of

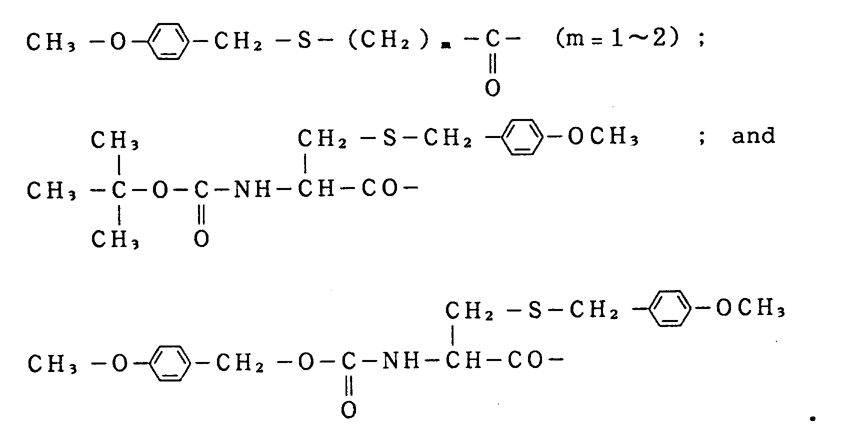

When the linker as mentioned above is added, as explained in Figure 1, the linker may couple with the mature target peptide alone, since only the mature peptide has the N-terminal amino group. Therefore, the active group included in the linker such as the SH group is introduced to only the mature peptide at the other terminus thereof.

According to the conventional method, the final deprotection reaction is effected under acidic conditions. The mature peptide coupled to the linker and the immature peptides end-capped, for example by an acetyl group, are cleaved from the solid-phase support. The linker is stable maintaining the bound state to the N-terminus of the mature peptide in the final deprotection reaction as explained before. When the compound represented by structural formula (III) is used as the linker, the p-methoxybenzyl group which is the protection group for the SH group of the linker is removed by the deprotecting reagent to allow the SH group to be exposed at the terminus. Then a ligand is allowed to contact with the above peptide mixture of mature and immature peptides. The ligand has a compound which has another functional group forming a stable bond with the functional group at the other end of the linker immobilized thereon. In the case where SH is the functional group, the ligand where iodo-substituted aliphatic carboxylic acid derivative or bromo-substituted aliphatic carboxylic acid derivative has been immobilized on the surface is used. Especially preferable is a derivative of iodo-substituted aliphatic carboxylic acid shown by the following general formula: 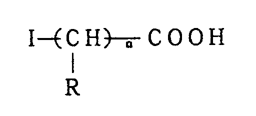
(wherein R is H or an alkyl group of C₁ to C₆, n is an integral of one to six);
or a derivative of bromo-substituted aliphatic carboxylic acid shown by the following general formula: 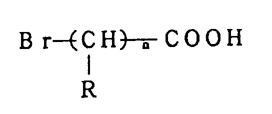
(wherein R is H or an alkyl group of C₁ to C₆, n is an integral of one to six).
Derivatives of iodoacetic acid I-CH₂COOH or bromoacetic acid BrCH₂COOH are more preferable.

Specifically, a ligand immobilizing the molecular chain having one of the structural formulae below is used, and it is subjected to contact with the mixture under near neutral conditions: 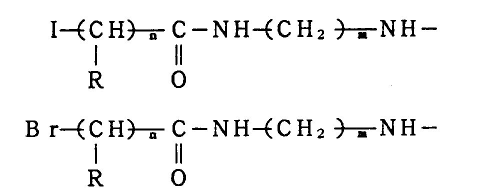
(wherein R is H or an alkyl group of C₁ to C₆, n=1 ∼ 2, m=2 ∼ 6.)
Preferably the reaction is allowed to proceed at around pH=7-9 and in an inert buffer so as not to disturb the expected reaction. More preferably, a denaturing agent such as guanidine hydrochloride or urea is added in order to increase the solubility of the peptides.

As an example, 0.4M phosphate buffer or 0.4M tris (hydroxymethyl)aminomethane hydrochloride buffer (abb., trishydrochloride buffer) is used. Guanidine hydrochloride of 4-7 mol, preferably ca.6M guanidine hydrochloride, or urea of 4-8M, preferably ca.6M urea is used as a denaturing agent. Other buffers and denaturing agents can be used on a case-by-case basis.

A solid phase support, especially a hydrophilic solid-phase support, is preferable as a ligand where a compound having these functional groups is immobilized. Kieselgur ligand (solid-phase support beads) to which a suitable hydrophilic solid-phase support is absorbed so that it is usable in an aqueous system is shown as an example.

By the above process, the selective immobilization of the mature peptide to the ligand can be attained. The next washing process of the ligand easily removes immature peptides which do not bond to the ligand and scavengers used for deprotection. The mature peptide is finally cleaved from the linker-bound ligand under such a specific condition that the bond between the linker and the mature peptide is selectively cleaved. For example, the mature peptide is cleaved from the linker-bound ligand under a basic condition, if the linker is a compound shown by structural formula (III). If 5% ammonium solution is used for this basic treatment, some peptides which are not dissolved in 5% NH₄OH may remain as precipitates in the column. If the 5% ammonium treatment is followed by an additional washing process with 50% acetic acid, the precipitates can be eluted from the column and recovered effectively. The washing of the ligand with acetic acid following the base treatment is also preferable from the view-point that ammonia is neutralized and may be sublimed by freeze-drying. Then the compound finally obtained is the mature peptide alone.

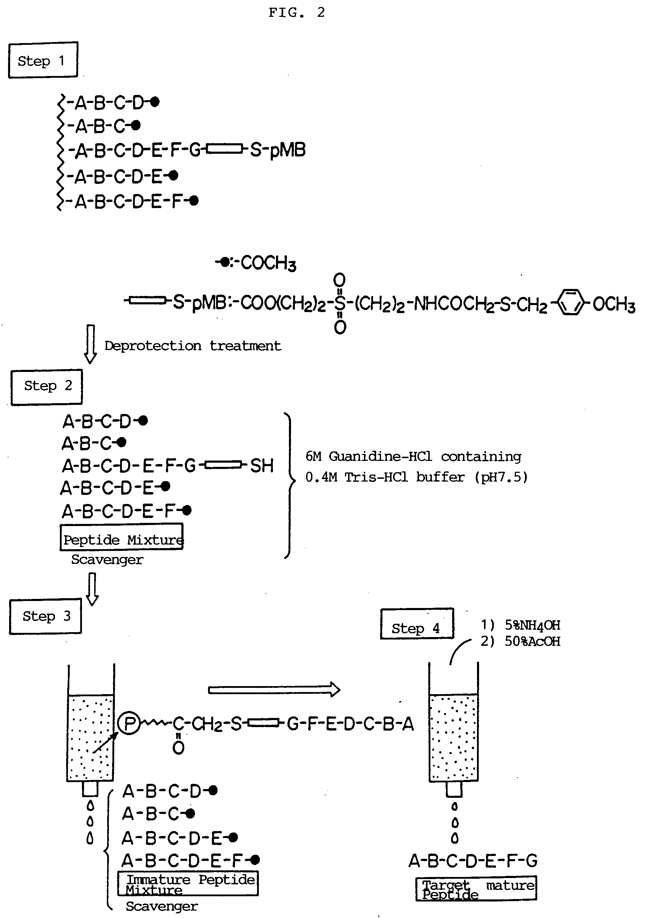

Figure 2 is a flow-chart showing one embodiment of the peptide purification method of the present invention. In this figure the mature target peptide is shown as A-B-C-D-E-F-G, and A-B-C, A-B-C-D, A-B-C-D-E, and A-B-C-D-E-F are impurities. Step 1 of Figure 2 shows the targeted mature peptide mixed with many impurities. Both the mature peptide having an N-terminal amino group and immature peptides (impurities) having an acetyl-group modified terminus by end-capping with acetic anhydride are bound to the solid-phase support (solid-phase support beads). The linker of the present invention as shown before is added to the solid-phase support, and the linker selectively bonds to the terminus of the mature peptide A-B-C-D-E-F-G alone. The final deprotection treatment in the peptide synthesis method cleaves the target peptide and immature peptides from the solid-phase support, as shown in step 2. This treatment as well allows the formation of an active functional group (SH group) at the other end of the linker bound to the target peptide. The next process shown in step 3 involves letting the mixture of the mature peptide and immature peptides to come in contact with a ligand of this invention packed in a column for bonding to the linker. The ligand bonds to the mature peptide alone via the linker, and immature peptides and other reagents are eluted from the column. Step 4 involves exposuring the column to the basic condition under which the bond between the linker and the mature peptide is selectively cleaved. This results in the cleavage of the mature peptide from the linker-bound ligand and the elution of the mature peptide from the column.

### Examples

The present invention is described more detail with a few experimental examples as below. However, it will be understood that the present invention is not limited to such a few disclosed examples.

### Example I: Synthesis of the linker (A)

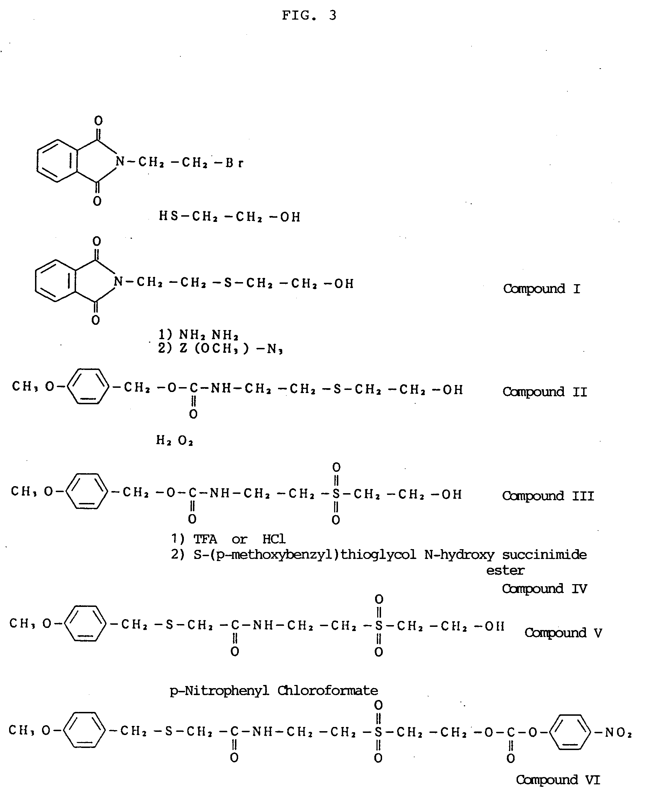

This example describes a synthesis method to obtain the linker, where the synthesis was made according to steps shown in Fig. 3

### 1-1 Synthesis of the compound (I)

### Compound (I):2-N-Ethylphthalimide, 2'-Hydroxyethylsulfide

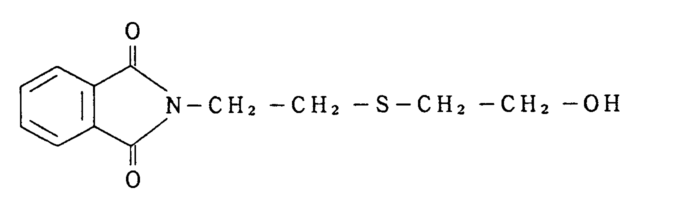

N-2-Bromoethylphthalimide (50.8g, 0.2mol) and β - mercaptoethanol (14.3ml, 0.2mol) were dissolved in dimethylformamide (400ml). After the further addition of dicyclohexylamine (39.9ml, 0.2mol) under cooling by ice, the solution was stirred for 4 hr. at room temperature. The produced salt (hydrobromide of dicyclohexylamine) was removed by filtration. The filtrate was mixed with the washing solution that was obtained by the washing of the salt with a small amount of dimethylformamide. It was followed by the evaporation of the dimethylformamide under reduced pressure. The residue was dissolved in ethyl acetate and transferred to a separatory funnel. The ethyl acetate phase was washed with saturated brine three times. The ethyl acetate phase, being transferred to a Mayer's flask, was dried over anhydrous sodium sulfate. Subsequent to the removal of sodium sulfate by filtration, ethyl acetate was evaporated under reduced pressure to obtain 45.9g of oily white compound (yield 91%).

### 1-2 Synthesis of compound (II)

### Compound (II): 2N-(p-methoxybenzyloxycarbonyl)aminoethyl, 2'-hydroxydiethylsulfide)

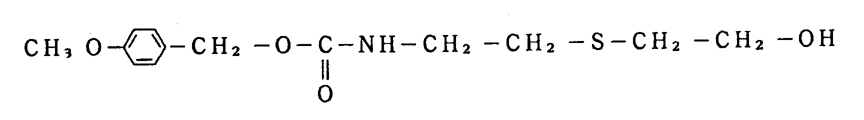

Compound I (43.5g, 0.17mol) obtained by the above procedure was dissolved in methanol (250ml). The solution was stirred for 16hr. after the addition of hydrazine hydrate (9.3ml, 0.18mol). The produced crystals were removed by the filtration, and subsequently methanol was evaporated under reduced pressure. The residue was crystallized by the addition of acetonitrile, and followed by the recrystallization from methanol/acetonitrile to obtain 45.9g of white crystals (yield 86%). The crystals were dissolved in a mixture of water (100ml) and triethylamine (23.6ml).

They are added to an acetonitrile solution (100ml) of parametoxy-benzylazidoformate (34.7g, 0.16mol from Watanabe Chemical Kogyo Ltd.) under cooling by ice, and then the solution was stirred for 10 hr. Subsequent to the evaporation of water/acetonitrile under reduced pressure, the oily residue was dissolved in ethyl acetate and transferred to a separatory funnel. The ethyl acetate phase was washed with 5% citric acid three times, and followed by washing 3 times with saturated brine. After the ethyl acetate phase was transferred into a Mayer's flask, it was dried over anhydrous sodium sulfate. Subsequent to the removal of sodium sulfate by filtration, ethyl acetate was evaporated under reduced pressure. To the residue n-hexane was added to obtain crystals. The crystals were further recrystallized by ethyl acetate/n-hexane to obtain 37.12g of white crystals (yield 93%).

The Specifications of Compound (II) above were as follows:
Rf₁ = 0.74
Melting point; 41 - 41.5 °C
The theoretical values of C₁₃H₁₉NO₄S:
C=54.72; H=6.71; N=4.91
The measured values:
C=54.79; H=6.88; N=4.98
NMR (CDCl₃): δ 2.08(br.s,1H)
2.73-2.76 (m,4H), 3.39(q,J=6. 1Hz, 2H), 3.73 (t, J=5.7Hz, 2H), 3.80 (s,3H), 5.04 (s, 2H), 5.12 (br. s, 1H) 6.88, 7.30 (AA' BB' pattern, Jₒᵣₜₕₒ=8.8).
FAB Mass Spectroscopy: 286.1 (M+H⁺)
(calculated on C₁₃H₁₉NO₄S: 285.1)

### 1-3 Synthesis of compound (III)

### Compound (III): N-(p-methoxybenzyloxy carbonyl) aminoethylsuphonyl ethanol

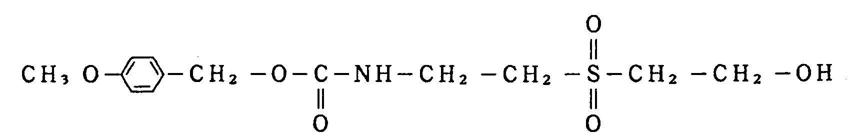

Compound II (18.2g, 65mmol) obtained by the above procedure was dissolved in water (100ml) and methoanol (100ml). After the addition of sodium tungstenate (65mg), hydrogen peroxide (16.3ml, 114ml/mol) was added dropwise to the solution while stirring. This was an exothermic reaction and the reaction temperature was controlled to 60 °C . After the two-hour stirring, additional stirring was made with the addition of 5% palladium/carbon (0.5g) until the foaming due to decomposition of excess hydrogen peroxide had ceased. 5% palladium/carbon was removed by filtration, and the solvent was evaporated under pressure. The oily residue was dissolved in ethyl acetate and transferred to a separatory funnel. The ethyl acetate phase was washed with 5% citric acid three times, 5% sodium bicarbonate one time and saturated brine three times. After the ethyl acetate phase was transferred into a Mayer's flask, it was dried over anhydrous sodium sulfate. Subsequent to the removal of sodium sulfate by filtration, ethyl acetate was evaporated under reduced pressure. To the oily residue diethyl ether was added to obtain crystals. The crystals were further recrystallized by ethyl acetate/diethyl ether to obtain 16.7g of white crystals (yield 81%).

The specifications of Compound (III) obtained by the above were as follows:
Rf₁ =0.52
Melting point 65 - 66 °C
The theoretical values of C₁₃H₁₉NO₆S:
C=59.20; H=6.03; N=4.41
The measured values:
C=59.03; H=5.97; N=4.41
NMR (CDCl₃): δ 2.09(br.s,1H),
3.20 (t, J=5. 4Hz, 2H), 3.34(t, J=5. 9Hz, 2H), 3.72 (q, J=5. 9Hz, 2H), 3.80 (s, 3H), 4.08 (t, J=5. 4Hz, 2H), 5.04 (s, 2H), 5.46 (br. t like, 1H), 6.88, 7.29 (AA' BB' pattern, Jₒᵣₜₕₒ =8.8).
FAB Mass Spectroscopy: 340.1 (M+Na⁺)
(calculated on C₁₃H₁₉NO₆SNa: 340.1)

### 1-4 : Synthesis of Compound IV

### Compound IV : S-(p-methoxybenzyl)thioglycolate N-hydroxy succinimide ester

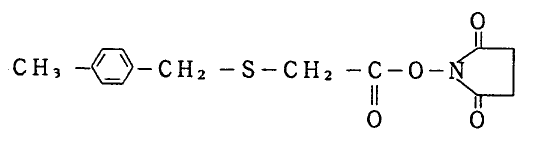

S-(p-methoxybenzyl)thioglycolic acid (1.49g, 7mmol) and 1-hydroxy succiimide (0.81g, 7mmol) were dissolved in tetrahydrofuran (10ml). After the further addition of N,N'-dicyclohexylcarbodiimide (1.59g, 7.7mmol) with ice cooling, the solution was stirred for 5 hr. The resultant N,N'-dicyclohexylurea was separated by filtration, and tetrahydrofuran was evaporated under reduced pressure. Isopropyl alcohol was added to the residue to obtain crystals. The crystals were further recrystallized from tetrahydrofuran/iso-propyl alcohol to obtain 1.25g of white crystals (yield 58%).

The specifications of compound (IV) obtained by the above were as follows:
Rf₂ =0.86
Melting point 88 - 89 °C
The theoretical values of C₁₄H₁₅NO₅S:
C=54.36; H=4.88; N=4.53
The measured values:
C=54.07; H=4.85; N=4.48
FAB Mass Spectroscopy: 332.1 (M+Na⁺)
(calculated on C₁₄H₁₅NO₅SNa: 332.1)

### 1-5 : Synthesis of Compound V

### Compound V : N[S-(p-methoxybenzyl)thioglycol aminoethyl sulphonylethanol

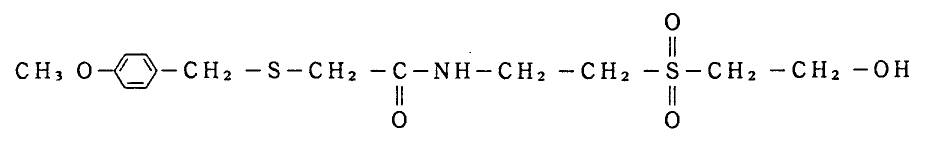

Compound III mentioned above (5.0g, 15.8mmol) was treated with trifluoroacetic acid (20ml) for 60 min. under the presence of anisol (5ml) with ice cooling. Trifluoroacetic acid was evaporated under reduced pressure. The residue was dissolved in diethyl ether to produce an oily residue. The oily residue dissolved in dimethylformamide (50ml) with ice cooling was stirred for 12 hr. at room temperature after the addition of triethyl amine (4.5ml), N-hydroxybenzotriazole (2.42g) and Compound IV (5.86g, 19.0mmol). Dimethylformamide was evaporated under reduced pressure to obtain the oily residue. The obtained oily residue was dissolved in ethyl acetate, and transferred a separatory funnel. The ethyl acetate phase was washed with 5% citric acid 3 times, 5% sodium bicarbonate 3 times, and saturated brine. The ethyl acetate transferred into a Mayer's flask was dried over anhydrous sodium sulfate. The removal of anhydrous sodium sulfate by a filtration was followed by the evaporation of ethyl acetate under reduced pressure. The obtained oily residue was crystallized by the addition of diethyl ether, and the obtained crystals were recrystallized from ethyl acetate/diethyl ether to obtain 4.31 g of white crystals (yield 78%).

The specifications of compound (V) obtained by the above were as follows:
Rf₁ =0.61
Melting point 73 - 75 °C
The theoretical values of C₁₄H₂₁NO₅S₂:
C=48.40; H=6.09; N=4.03
C=48.40; H=6.09; N=4.03
The measured values:
C=48.60; H=6.29; N=4.11
NMR (CDCl₃): δ 2.29 (br.s,1H),
3.13 (s, 2H), 3.31 - 3.23 (m, 4H), 3.71 (s, 2H), 3.72 (q, J=6. 0Hz, 2H), 3.80 (s, 3H), 4.13 (t, J=5. 2Hz, 2H), 6.86, 7.22 (AA' BB' pattern, Jₒᵣₜₕₒ=8.7) 7.31 (br. t like, 1H).
FAB Mass Spectroscopy: 348.1 (M+H⁺)
(calculated on C₁₄H₂₁NO₅S₂: 347.1)

### 1-6 : Synthesis of Compound VI (linker)

### Compound VI :N-[S-(p-methoxybenzyl)thioglycolyl] aminoethylsulphonylethyl p-nitrophenylcarbonate

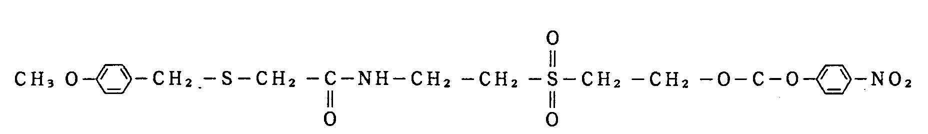

The compound V mentioned before (4.00g, 11.5mmol) was dissolved in anhydrous pyridine (30ml). After the addition of para-Nitrophenyl chloroformate (2.32g, 11.5mmol) under ice cooling, the solution was stirred for 4hr. Pyridine was evaporated under reduced pressure. The produced oily residue was crystallized by the addition of 1N hydrochloric acid (50ml) and diethyl ether. The obtained crystals are recrystallized from hydrochloric acid/diethyl ether to obtain 4.07g of white crystals (yield 69%).

The specifications of compound (VI) obtained by the above were as follows:
Rf₂ =0.37
Melting point 81 - 82 °C
The theoretical values of C₂₁H₂₄N₂O₉S₂:
C=49.21; H=4.72; N=5.47
The measured values:
C=49.30; H=4.72; N=5.25
NMR (CDCl₃): δ 3.13 (s, 2H),
3.28 (t, J=6. 0Hz, 2H), 3.47 (t, J=5. 7Hz, 2H), 3.75 (q, J=6. 0Hz, 2H), 3.79 (s, 3H), 4.74 (t, J=5. 7Hz, 2H), 6.84, 7.20 (AA' BB' pattern, Jₒᵣₜₕₒ=8.7) 7.25 (m, 1H), 7.40, 8.28 (AA' BB' pattern, Jₒᵣₜₕₒ=9.3).
FAB Mass Spectroscopy: 513.2 (M+H⁺)
(calculated on C₂₁H₂₄N₂O₉S₂: 512.1)

### Example II: Synthesis of the linker (B)

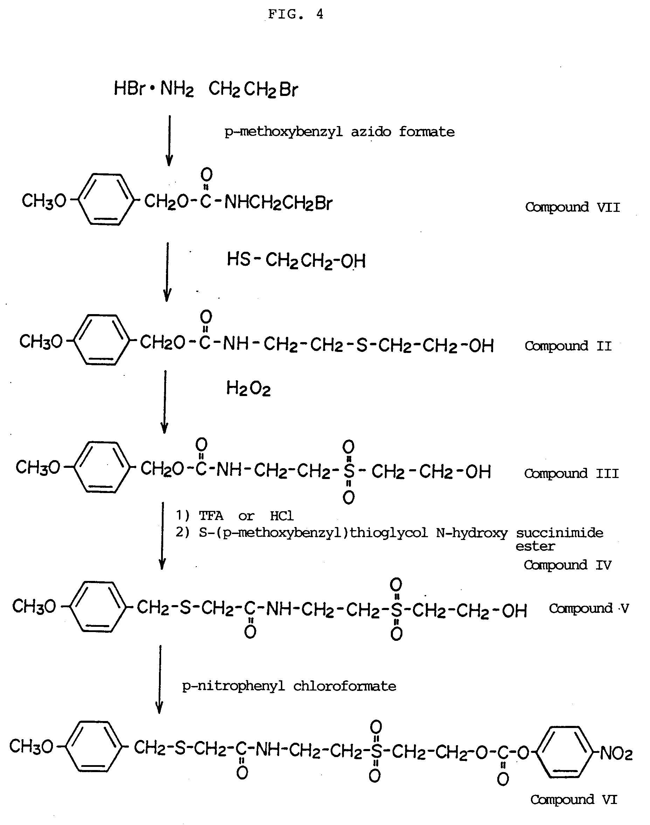

This example describes another synthesis of the linker, according to steps shown in Fig. 4.

### 2-1 Synthesis of the compound (VII)

### Compound (VII) : N-(p-methoxybenzyloxycarbonyl)aminoethyl bromide

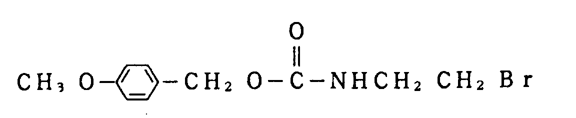

Hydrobromide of 2-bromoethylamine (100.0g, 0.49mol) was dissolved in tetrahydrofuran (THF 500ml). After the further addition of para-methoxybenzylazido formate (111.2g, 0.54mol) and triethylamine (148.9ml, 1.07mol) under ice cooling, the solution was stirred for 10 hr. The produced salt (hydrobromide of triethylamine) was removed by a filtration. The filtrate was mixed with the washing solution that was obtained by the washing of the salt with a small amount of THF. The evaporation of THF under reduced pressure was followed. The residue was dissolved in ethyl acetate and transferred to a separatory funnel. Ethyl acetate phase was washed with 5% citric acid three times and with saturated brine three times. The ethyl acetate phase, being transferred to a Mayer's flask, was dried over anhydrous sodium sulfate. Subsequent to the removal of anhydrous sodium sulfate by filtration, ethyl acetate was evaporated under reduced pressure. The residue was crystallized by the addition of n-hexane followed by recrystallization from ethyl acetate/n-hexane to obtain 102.5g of white crystals (yield 73%).

The specifications of Compound (VII) above were as follows:
Rf₄ =0.3
Melting point 44.5 - 45.5 °C
The theoretical values of C₁₁H₁₄NO₃Br:
C=45.85; H=4.90; N=4.86
The measured values:
C=45.59; H=4.84; N=4.80

### 2-2 Synthesis of Compound (II)

Compound VII (100g, 0.35mol) obtained by the above method and β -mercaptoethanol (25.0ml, 0.35mol) were dissolved in dimethylformamide (DMF, 700ml). After the further addition of dicyclohexylamine (70ml, 0.35mol) under ice cooling, the solution was stirred for 4 hr. at room temperature. The produced salt (hydrobromide of dicyclohexylamine) was removed by filtration. The residue was mixed with washing solution obtained by the washing of the bromide with a small amount of DMF. DMF was evaporated under reduced pressure. The residue dissolved in ethyl acetate, was transferred to a separatory funnel. The ethyl acetate phase was washed with 5% citric acid three times, and followed by saturated brine three times. The ethyl acetate phase was transferred to a Mayer's flask, and subsequently ethyl acetate was dried over anhydrous sodium sulfate. Subsequent to the removal of anhydrous sodium sulfate by filtration, ethyl acetate was evaporated under reduced pressure. N-hexane is added to the residue for crystallization. The obtained crystals were further recrystallized from ethyl acetate/n-hexane to obtain 90.5g of white crystals (yield 91%). The melting point of compound II obtained was 41 - 41.5 °C .

### 2-3 Synthesis of compound (VI) (linker)

Compound (III) was produced from Compound II, obtained as above, according to the procedure described in 1-3. Compound V was synthesized according to the procedure described in 1-5 and compound VI (linker) was produced according to the procedure in 1-6.

### Example III : Synthesis of the linker (C)

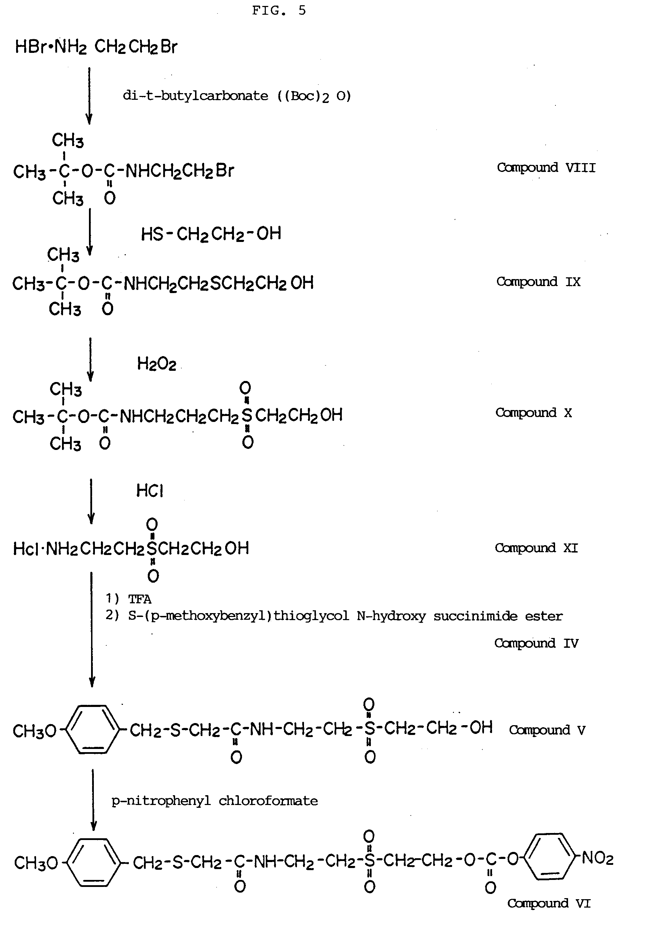

This example describes another method for the synthesis of the linker, according to steps shown in Fig. 5.

### 3-1 Synthesis of the compound (VIII)

### Compound (VIII) : N-(t-butyloxycarbonyl)aminoethyl bromide

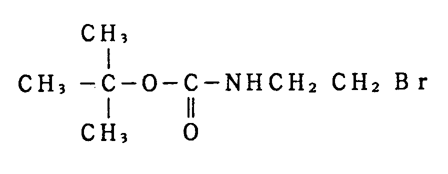

Hydrobromide of 2-Bromoethylamine (10g, 0.49mmol) was dissolved in dimethylformamide (DMF 100ml). After the further addition of di-t-butyldicarbonate ((Boc)₂0) (11.8g, 54mmol, 1.1 equivalent) and triethylamine (10.3ml, 74mmol, 1.5 equivalent) under ice cooling, the solution was stirred for 2 hr. at room temperature. The produced salt (hydrobromide of triethylamine) was removed by filtration, The filtrate was mixed with the solution obtained by washing the salt with a small amount of DMF. Rf₂ of this compound is 0.90.

### 3-2 Synthesis of Compound IX

### Compound (IX) : 2N-(t-butyloxycarbonyl)aminoethyl, 2'-hydroxyethylsulfide

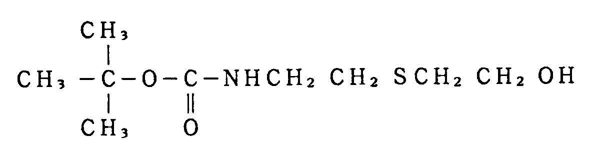

DMF solution (filtrate and washing solution) of Compound VIII (49mmol) obtained above was mixed with β -mercaptoethanol (3.5ml). After the further addition of dicyclohexylamine (9.8ml, 49mmol) under ice cooling the solution was stirred for 20 hr. at room temperature. The produced salt (hydrobromide of dicyclohexylamine) was removed by filtration. The filtrate was mixed with the solution obtained by the washing of the salt with DMF. DMF was evaporated under reduced pressure. The residue was dissolved in ethyl acetate, and the ethyl acetate solution was washed with 5% citric acid three times and with saturated brine three times. The washed ethyl acetate slution is dried over anhydrous sodium sulfate. Subsequent to the removal of anhydrous sodium sulfate by filtration, ethyl acetate was evaporated under reduced pressure to obtain a transparent oily compound (Compound IX).

### 3-3 Synthesis of compound (X)

### Compound X : N(t-butyloxycarbonyl)aminoethylsulphoethanol (oily compound)

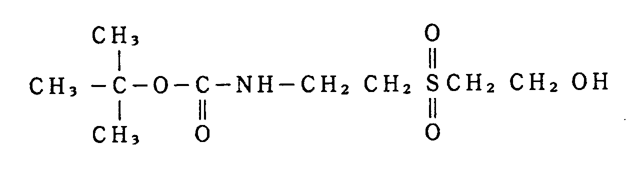

Compound (IX) (49mmol) obtained by the procedure described in 3-2 was dissolved in methanol (85ml). Water (40ml) followed by sodium tungstate (49mg) were added, and hydrogen peroxide (11.3ml) was added dropwise with stirring of the solution. This was an exothermic reaction and the reaction temperature was controlled to remain below 60 °C. After stirring for one-hour and a half 5% paladium/carbon (0.38g) was added with additional stirring to decompose the excess hydrogen peroxide. The stirring was continued until the foaming had ceased. 5% paladium/carbon was removed by filtration, and the filtrate was evaporated under reduced pressure. The oily residue, dissolved in ethyl acetate, was washed with 5% citric acid three times, 5% sodium hydrogencarbonate one time and saturated brine three times. After the ethyl acetate phase was transferred to a Mayer's flask, it was dried over anhydrous sodium sulfate. Subsequent to the removal of anhydrous sodium sulfate by filtration, ethyl acetate was evaporated under reduced pressure 11.79g of oily compound (compound X) was obtained.

### 3-4 : Synthesis of Compound XI

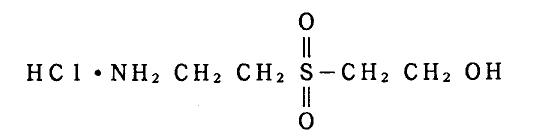

### Compound XI : Aminoethylsulphonylethanol hydrochlorate

Compound X (49mmol) obtained by the procedure described above in 3-3 was dissolved in 4N-HCl/dioxane (40ml) and stirred for 30-min. The obtained crystals were collected by filtration and washed with ether. The crystals were subsequently dried under vacuum. 6.6g (35mmol) of crystals (Compound XI) were obtained, and the yield was 71%.
Rf₅ =0.45
Melting point 255 °C
The theoretical values of C₄H₁₂NO₃SCl₁₂:
C=25.33; H=6.38; N=7.39
The measured values:
C=25.24; H=6.50; N=7.43

### 3-5 : Synthesis of Compound V

Compound V was synthesized from Compound XI, produced by the above method, according to the procedure described in 1-5.

### 3-6 : Synthesis of Compound VI (linker)

Compound VI (linker) was synthesized from compound V mentioned above according to the procedure described in 1-6.

### Example 4 : Synthesis of Resin for bonding the Linker Synthesis of Iodo Acetic acid resin

Methylene chloride solution (50ml) of iodo acetic acid (7.4g, 40mmol), where dicyclohexylcarbodiimide (5g, 0.6eq.) was added, was stirred for 30 min. under ice cooling. The produced preticipate (dicyclehexylurea) was removed by filtration. The filtrate was treated with ethylenediamine. Followingly the treated filtrate was added to PepSyn K resin * whose terminal aminoethyl group was attached (20g, amino group content: 0.2mmol/g), and shaked till Kaiser test indicated negative (2 hr). The resin was washed well with dimethyformamide and Methylene chloride, and dried under vacuum.
*produced by Millipore Co., Ltd., Kieselgur type resin: covalent polymer of dimethylacrylamide, bis acroylethylenediamine and acryloylsarcosine methylester was supported by Kieselgur

### Example 5 : Purification of Synthesized Peptide (1)

Polyphemusin II was synthesized and purified in order to clarify the availability of this purification of the present invention. Polyphemusin II was synthesized by Fmoc-based solid-phase synthesis according to the procedure shown in Fig. 1, and purified, Polyphemusin II is a 18-residue peptide with C-terminus amide and two disulfide bonds. For the synthesis, cys derivative whose thiol group was protected with Acm (acetamide methyl) was used. 
H-Arg-Arg-Trp-Cys(Acm)-Phe-Arg-Val-
Cys(Acm)-Tyr-Lys-Gly-Phe-Cys(Acm)-
Tyr-Arg-Lys-Cys(Acm)-Arg-NH2
At the final step of the synthesis, linker prepared according to Example 1 (512mg, 5 equ.) and N-Hydroxybenzotriazole (152mg, 5 equ.) were added to the solid phase resin (0.2mmol), and then stirred in Dimethylformamide (10ml) for 2 hr. in order to attach the linker to the N-terminus of Polyphemusin synthesized on the resin. Subsequently the deprotection of the solid phase resin was carried out at 0 °C for 2 hr. with a solution (10ml) of 1M trimethylsilyl bromide (TMSBr)-thioanile/trifluoroacetic acid (TFA) in the presence of m-cresol (0.5ml) [N.Fujii, A.Otaka, N.Sugiyama, M.Hatano, and H.Yajima, Chem. Pharm. Bull., 35, 3880]. TMSBr and TFA were evaporated off, and diethyl ether was added to obtain the peptide as a powder. The obtained powder was dissolved in 0.4M Tris hydrochloric acid buffer (pH7.5) containing 6M guanidine hydrochloride. Subsequently the solution was introduced onto a column packed with 1 equivalent of iodo acetic acid resin prepared by the procedure described in Example 4., and it was recirculated for 2 hr. to allow reaction. After the treatment with 1 equ. of 2-mercaptoethanol for 2 hr., excess iodo acetic acid resin was washed well with 0.4M tris hydrochloric acid buffer (pH7.5) containing 6M guanidine hydrochloride, 5.0% acetic acid, and water sequentially.

The obtained peptide-bound resin was treated with 5% ammonium hydroxide solution for 30 min. to cleave the peptide from the resin. After the further washing of the resin with 50% acetic acid, the wash solution was added to the above reagent for cleaving. The obtained liquid was freeze-dried to obtain 14.2mg of the peptide powder. Analysis by HPLC showed a single peak (purity: >99%).

The HPLC analysis of crude Polyphemusin II just after the solid-phase synthesis was ca.69%, and a shoulder peak before the main peak was observed. However, the purified peptide obtained by the method of the present invention had a single peak. The results of amino acid sequence analysis and FAB mass spectroscopy of the purified peptide agreed with the theoretical [Acm-Polyphemusin II:2714.1 (M+H⁺), calculated on C_{120̸}H₁₈₅N₄₁O₂₄S₄].

### Example 6: Purification of Synthesized Peptide (2)

The present invention was employed for the synthesis of human cholecystokinin, a small sized protein in order to clarify the availability of this purification.

Human cholecystokinin was synthesized by Fmoc-based solid-phase synthesis according to the procedure shown in Fig. 1, and purified. Human cholecystokinin is a 33-residue Human cholecystokinin is a 33-residue peptide. 
H-Lys-Ala-Pro-Ser-Gly-Arg-Met-Ser-
Ile-Val-Lys-Asn-Leu-Gln-Asn-Leu-Asp-
Pro-Ser-His-Arg-Ile-Ser-Asp-Arg-Asp-
Tyr-Met-Gly-Trp-Met-Asp-Phe-NH2
At the final step of the synthesis, linker prepared according to Example 1 (512mg, 5 equ.) and N-Hydroxybenzotriazole (152mg, 5 equ.) were added to the solid phase resin (0.2mmol), and subsequently stirred in dimethylformamide (10ml) for 2 hr. in order to attach the linker to the N-terminus of human Cholecystokinin synthesized on the resin. Deprotection was carried out at 0 °C for 2 hr. by 1M TMSBr-thioanile/Trifluoroacetic acid (TFA) (10ml) in the presence of ethanediol (0.2ml) and m-cresol (0.5ml). Following the deprotection, TMSBr and TFA were evaporated off, and diethylether was added to obtain the peptide as a powder. The obtained powder was dissolved in 0.4M Tris hydrochloric acid buffer (pH7.5) containing 6M guanidine hydrochloride. Subsequently the powder was introduced onto a column, where 1 equ. of iodo acetic acid resin prepared by the procedure in Example 4 is packed, to allow recirculation for 2 hr.. By the treatment with 1 equ. of 2-mercaptoethanol of the resin for 2 hr., excess iodo acetic acid was deactivated. After the removal of the solution from the column, the resin was washed well by 0.4M tris hydrochloric acid buffer (pH7.5) containing 6M guanidine hydrochloride, 5.0% acetic acid, and water sequentially.

The obtained peptide-bound resin (ligand) was treated with 5% ammonium hydroxide solution for 30 min. to cleave the peptide from the resin. After the further washing of the resin with 50% acetic acid, the wash solution was added to the above reagent for cleaving. The obtained liquid was freeze-dried to obtain 7.9mg of the peptide powder. Analysis by HPLC showed a single peak (purity : more than 90%). The purity of human cholecystokinin of non-sulphuric acid was 29%, when calculated from crude peptide just after synthesis by the Fmoc method. The retention time by HPLC of non-sulphuric acid cholecystokinin purified by the method of this invention was identical to that purified by HPLC. The results of amino acid sequence analysis and FAB mass spectroscopy of the purified peptide agreed with the theoretical [:3865.2 (M+H⁺), calculated on C₁₆₇H₂₆₃N₅₁O₄₉S₃].

### Example 7 : Purification of Synthesized Peptide (3)

The present invention was employed for the synthesis of another small sized protein, human Growth hormone Releasing Factor (hGRF), in order to clarify the availability. hGRF was synthesized by Fmoc-based solid-phase synthesis according to the procedure shown in Fig. 1, and purified. hGRF is a 44-residue peptide. 
H-Tyr-Ala-Asp-Ala-Ile-Phe-Thr-Asn-

Ser-Tyr-Arg-Lys-Val-Leu-Gly-Gln-Leu-

Ser-Ala-Arg-Lys-Leu-Leu-Gln-Asp-Ile-

Met-Ser-Arg-Gln-Gln-Gly-Glu-Ser-Asn-

Gln-Glu-Arg-Gly-Ala-Arg-Ala-Arg-Leu-NH2
At the final step of the synthesis, the linker prepared according to Example 1 (512mg, 5 equ.) and N-Hydroxybenzotriazole (152mg, 5 equ.) were added to the solid phase resin (0.2mmol), and subsequently stirred in dimethylformamide (10ml) for 2 hr. in order to attach the linker to the N-terminus of hGF synthesized on the resin. Deprotection was carried out at 0 °C for 2 hr. with 1M TMSBr-thioanile/trifluoroacetic acid (TFA) (10ml) in the presence of ethanediol (0.2ml) and m-cresoi (0.5ml). Following the deprotection, TMSBr and TFA were evaporated off, and diethylether was added to obtain the peptide as a powder. The obtained powder was dissolved in 0.4M Tris hydrochloric acid buffer (pH7.5) containing 6M guanidine hydrochloride. Subsequently the powder was introduced on to a column, where 1 equ. of iodo acetic acid resin prepared in Example 4 was packed, to allow recirculation for 2 hr.. By the treatment with 1 equ. of 2-mercaptoethanol of the resin for 2 hr., excess iodo acetic acid resin was endcapped. After the removal of the solution from the column, the resin was washed well with 6M .5), 5.0% acetic acid, and water sequentially.

The obtained peptide-bound resin (ligand) was treated with 5% ammonium solution for 30 min. to cleave the peptide from the resin. After the further washing of the resin with 50% acetic acid, the wash solution was added to with reagent for cleaving. the obtained liquid was freeze-dried and 7.9mg of the peptide powder was obtained. Analysis by HPLC showed almost a single peak (purity : <80%). The purity of hGF was 51%, when calculated from crude peptide just after synthesis by the Fmoc method. The retention time by HPLC of hGF purified by the method of this invention was identical to that purified by HPLC.

The results of amino acid sequence analysis and FAB mass spectroscopy of the purified peptide agreed with the theoretical [5037.8 (M+H⁺), calculated on C₂₁₅H₃₅₈N₇₂O₆₆S].

Instruments and Reagents used for assay of the above examples are as follows:

### Thin Layer Chromatography

Sorbent : silica gel G (Kiesel gel G, E.Merck, Germany)
Solvents :
Rf₁ : Lower phase of CHCl₃-MeOH-H₂O (8:3:1)
Rf₂ : CHCl₃-MeOH (10:0.5)
Rf₃ : CHCl₃-AcOH (19:1)
Rf₄ : CHCl₃-Cyclohexane (2:1)
Rf₅ : n-BuOH-AcOH-Pyridine-H₂O (4:1:1:2)

### Nuclear Magnetic Resonance

Bruker AC-300 spectorometer (Bruker Co., Ltd.)
Inner standard : tetramethylsilane

### FAB Mass spectrocopy

ZAB SE Mass spectrometer (U.G. Anlytical)

### Purity Analysis of peptide

### 1) High Performance Liquid Chromatograph (HPLC)

HPLC system Model 600E (Millipore Co.,)
µ -Bondasphere 5C18 column (Millipore Co.,)
with solvent system of water (0.1% trifluoro acetic acid)- acetonitrile

### 2) Amino acid sequence of peptides

Peptide sequencer Model 431A (Applied Biosystems Co.) and protein sequencer Model 6600 (Millipore Co.,)

### Synthesis of model peptides

Fmoc peptide synthesis by automated peptide synthesizer Model 9050 (Millipore Co.,)

## Claims

1. A method for purifying a peptide synthesized by a solid-phase method comprising:
applying a linker having a functional group at a terminus to a mixture system of a mature peptide having an amino group at a terminus and of immature end-capped peptides, thereby immobilizing chemical-selectively to a solid-phase support and separating said mature peptide from said mixture.

2. The method according to claim 1 further comprising:
a) adding said linker to a first solid-phase to which both said target mature peptide having the amino group at the terminus and said immature end-capped peptide have been bound after addition of the final amino acid for the solid-phase peptide synthesis process, thereby selectively modifying said mature peptide with said linker, wherein said linker has at one terminus a functional group which is able to form a bond with the N-terminus of said mature peptide, said bond being stable under the following cidic deprotection reaction condition performed as a post process of peptide synthesis and being specifically cleaved under another specific condition, and wherein the other terminus site of said linker is able to be converted into another active functional group by the deprotection reaction; and
b) performing the deprotection reaction as the final process of peptide synthesis, thereby dissocianating said mature peptide and immature peptides from said first solid-phase support;
c) putting the resultant freed peptide mixture of above b) step into contact with a second solid-phase ligand, thereby immobilizing selectively said mature peptide to said second solid-phase via said linker, wherein said ligand has a compound immobilized thereon and which having a functional group forming stable bond with the functional group at said terminus of said linker;
d) exposing the mature peptide immobilized ligand of above c) step to said other condition under which the bond between said linker and said mature peptide is selectively cleaved, thereby separating said mature peptide from the linker-bound ligand.

3. The method according to claim 1 comprising:
a) adding said linker to a first solid-phase to which both said mature target peptide having the amino group at the terminus and said immature end-capped non-target peptides have been bound after the final amino acid addition of the solid-phase peptide synthesis process, thereby selectively modifying said mature peptide with said linker, wherein said linker has at one terminus a functional group which is able to form a bond with the N-terminus of said mature peptide, said bonding being stable under the following acidic deprotection reaction condition and being a specifically cleaved under a basic condition, and wherein the other terminus site of said linker is able to be converted into an SH group by the deprotection reaction :
b) performing the deprotection reaction as the final process of peptide synthesis, thereby dissocianating of said mature peptide and immature peptides from said first solid-phase support;
c) putting the resultant freed peptide mixture of above b) step into contact with a second solid-phase ligand , thereby immobilizing selectively said mature peptide to said second solid-phase via said linker, wherein said ligand has a compound immobilized thereon and which having a functional group forming a stable bond with the SH group at said terminus of said linker;
d) exposing the mature peptide immobilized ligand of above c) step to said basic condition under which the bond between said linker and said mature peptide is selectively cleaved, thereby separating said mature peptide from the linker-bound ligand.

4. The method of claim 1 further comprising:
a) adding said linkder to a first solid-phase to which both said mature target peptide having the terminal amino group and said immature end-capped non-target peptides have been bound after the final acid addition of the solid-phase peptide synthesis process, thereby selevtively modifying said mature peptide with said linker, wherein said linkder has at one terminus an alkoxycarbonate bond which is able to form a urethane bond with the N-terminus of said mature peptide, said urethane bond being stable under the following acidic deprotection reaction condition and being a specifically cleaved under a basic condition, and wherein the other terminus site of said linker is able to be converted into an SH group by the deprotection reaction;
b) performing the deprotection reaction as the final process of peptide synthesis, thereby dissocianating said mature peptide and said immature peptides from said solid-phase support, while cleaving a thiol protecting group existing at the other terminus of said linker bound to said mature peptide to produce said thiol group at said terminus of said linker;
c) putting the resultant peptide mixture of above b) step into contact with a second solid-phase ligand under a neutral condition, wherein said ligand has a compound selected from the group consisting of iodo- and bromo- substituted aliphatic carboxylic acids derivatives and immobilized thereon, thereby selectively immobilizing said mature peptide to said ligand via said linker with a stable covalent bond, the covalent bond resulting from the deiodo- or debromo- reaction; and
d) exposing the mature peptide immobilized ligand of above c) step to said basic condition, wherein beta-elimination reaction is allowed to affect the bond between said linker and said mature peptide, thereby cleaving said mature peptide from the linker-bound ligand.

5. The method of claim 1 and 4 wherein said linker has chemical structure (III): 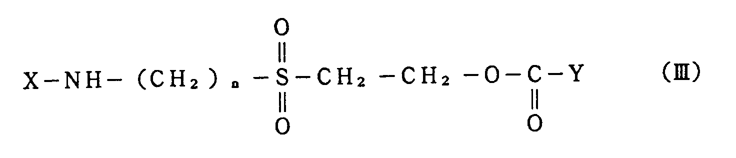 wherein n is an integral, X is one of 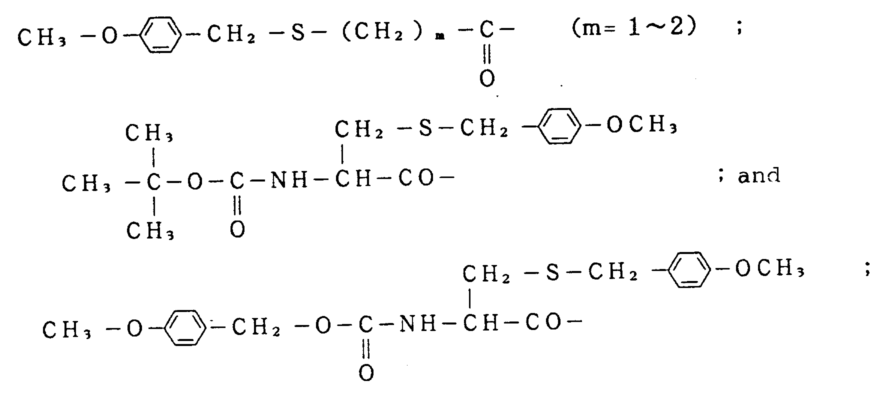 Y is one of

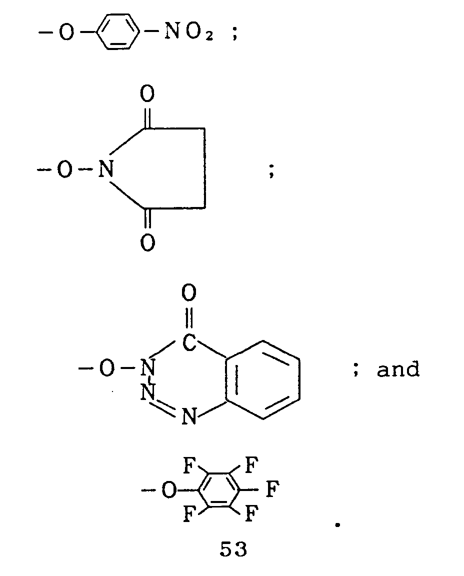

6. The method of claim 4 wherein said ligand used in the c) step has a derivative from a compound selected from the group consisting of iodo-aliphatic carboxylic acids having the following general formula: 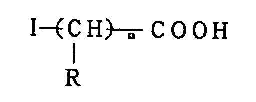 (wherein R is H or an alkyl group of C₁ ∼ C₆, n is an integral of 1 ∼ 6) and bromo-aliphatic carboxylic acids derivatives having the following general formula: 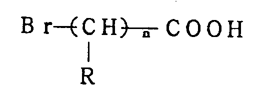 (wherein R is H or an alkyl group of C₁ ∼ C₆, n is an integral of 1 ∼ 6), and said compound being immobilized on said ligand.

7. The method of claim 4 wherein said ligand used in the c) step has a derivative from a compound selected from the group consisting of ω -iodo-aliphatic carboxylic acids having the following general formula: 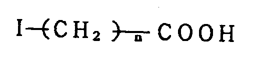 (wherein n=1 ∼ 6) and ω -bromo-aliphatic carboxylic acids having the following general formula: 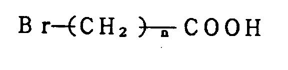 (wherein n=1 ∼ 6), and said derivative being immobilized on said ligand.

8. The method of claim 4 wherein said ligand used in the c) step has a derivative from I-CH₂COOH and Br-CH₂COOH, and said derivative being immobilized on said ligand.

9. The method of claim 4 wherein said ligand used in the c) step has a molecular chain described by the following chemical formula and bound on the surface of said ligand; 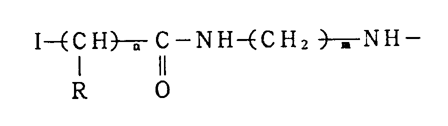 (wherein n=1 ∼ 6, m=1 ∼ 6, R is H or an alkyl group of C₁ ∼ C₆ )

10. The method of claim 4 wherein said ligand used in the c) step has a molecular chain described by the following chemical formula and bound on the surface of said ligand; 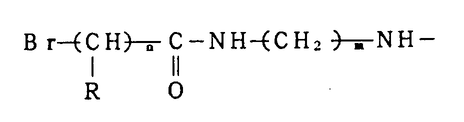 (wherein n=1 ∼ 6, m=1 ∼ 6, R is H or an alkyl group of C₁ ∼ C₆ )

11. The method of claim 4 wherein said ligand used in the c) step has a hydrophilic surface where a derivative from a compound selected from the group consisting of iodo-aliphatic carboxylic acids and bromo-aliphatic carboxylic acids have been bound.

12. The method of claim 4 wherein said ligand used in the c) step is a kieselguhr support usable in hydrophilic systems, wherein said kieselguhr support has an absorbed hydrophilic polymer containing an immobilized derivative from a compound selected from the group consisting of iodo-aliphatic carboxylic acids and bromo-aliphatic carboxylic acids.

13. A linker represented by chemical structure (III) as below: 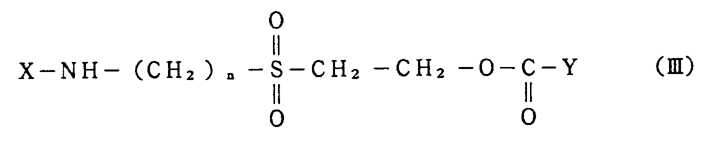 wherein n is an integral of one to four,
X is one of 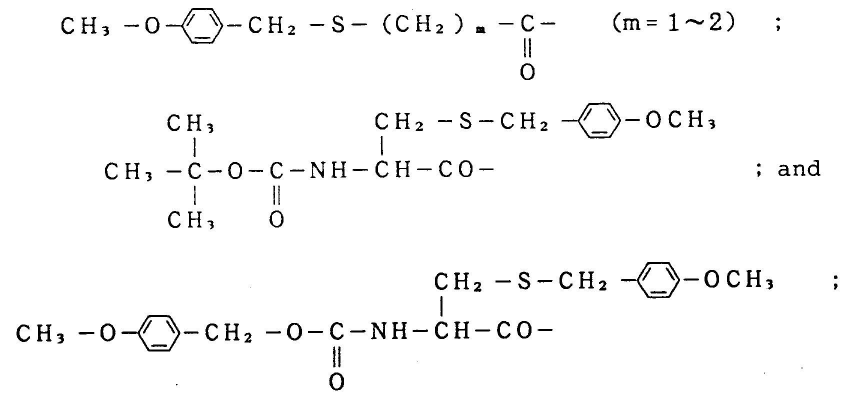 Y is one of

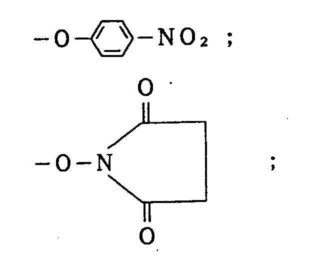

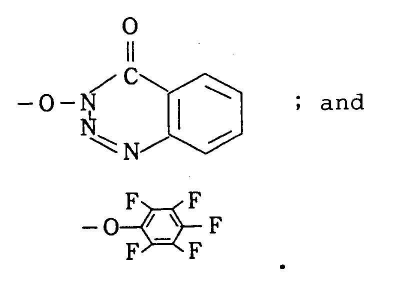

14. A intermediate for synthesising linker which is represented by chemical structure (I) as below: 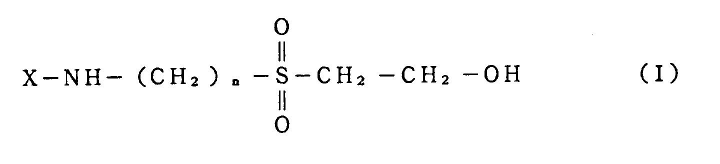 wherein n is an integral of one to four, X is one of H, hydrochloride thereof,

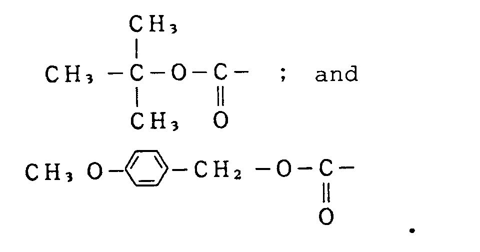

15. A intermediate for synthesising linker which is represented by chemical structure (II) as below : 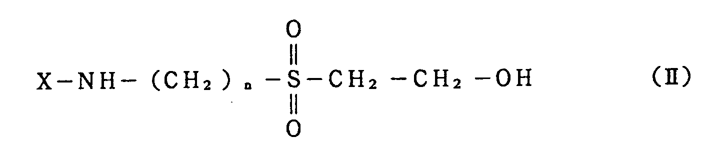 (wherein n is an integral of one to four,
X is one of

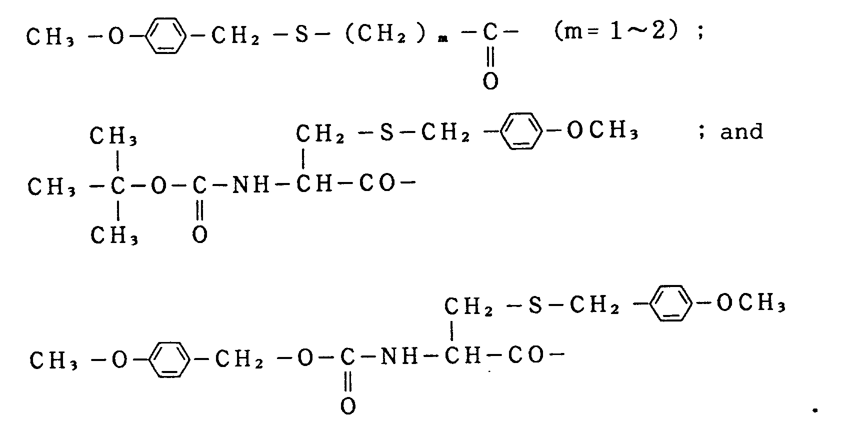

16. The linker of claim 13 wherein said linker is N-[S-(p-methoxybenzyl)thioglycolyl]aminoethylsulphonylethyl p-nitorphenyl-carbonate represented by the chemical structure as below:

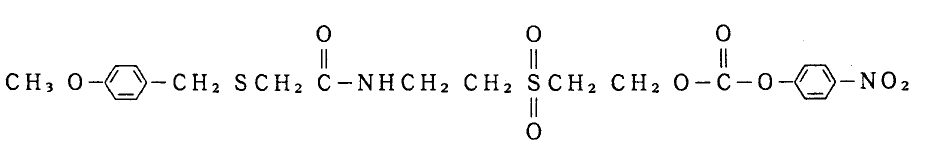

17. A new compound of N-(p-methoxybenzyloxycarbonyl) aminoethyl-bromide comprising a chemical structure as below:

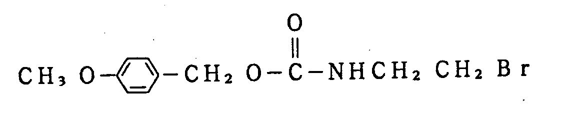

18. A new compound of N-(t-butyloxycarbonyl)aminoethylbromide comprising a chemical structure as below:

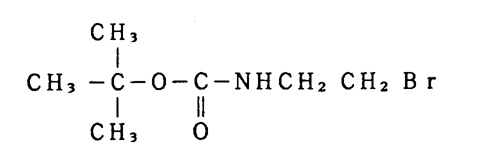

19. A new compound of 2-(aminoethyl sulphonyl)ethanol comprising a chemical structure as below (confirmed as hydrochloride thereof):

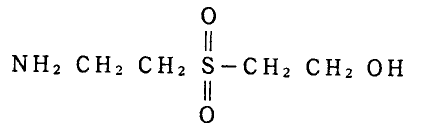

20. A new compound of 2-(aminoethyl sulphonyl)ethanol hydrochloride comprising a chemical structure as below:

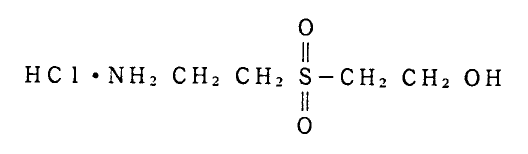

21. A ligand for bonding linker comprising having a molecular chain represented as below immobilized on the surface thereof: 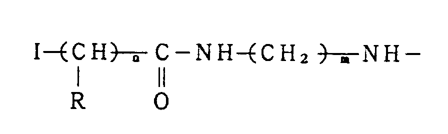 (wherein n=1 ∼ 6, m=1 ∼ 6, R is H or an alkyl group of C₁ ∼ C₆ )

22. A ligand for bonding linker comprising having a molecular chain represented as below immobilized on the surface thereof: 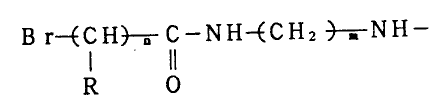 (wherein n=1 ∼ 6, m=1 ∼ 6, R is H or an alkyl group of C₁ ∼ C₆ )

23. The ligand for bonding linker of claim 21 or 22, wherein said ligand immobilizing said molecular chain is a hydrophilic resin.

24. The ligand for bonding linker of claim 21 or 22, wherein said ligand has an absorbed hydrophilic resin and is usable in aqueous systems.

25. The ligand for bonding linker of claim 24, wherein said ligand is a kieselguhr support which has a hydrophilic resin absorbed thereto and is usable in the hydrophilic solvent systems.
